# EUROPEAN PATENT SPECIFICATION

(11) **EP 4 288 411 B1**
(45) Date of publication and mention of the grant of the patent: **05.11.2025**
(21) Application number: 22703567.2
(22) Date of filing: 24.01.2022
(51) Int. Cl.: C07C 229/24, C07C 229/26, C07C 229/76, C07C 237/10, C07C 237/12

(54) **SOLUBLE CHELATOR FOR TARGETING RECOMBINANT PROTEINS**
LÖSLICHER CHELATBILDNER ZUM TARGETING REKOMBINANTER PROTEINE
CHÉLATEUR SOLUBLE POUR LE CIBLAGE DE PROTÉINES RECOMBINANTES

(30) Priority: 02.02.2021 US 202163144907 P
(43) Date of publication of application: 13.12.2023
(73) Proprietor: Cube Biotech GmbH, 40789 Monheim (DE)
(72) Inventor: FABIS, Roland, 51375 Leverkusen (DE); KUBICEK, Jan, 50933 Köln (DE); MAERTENS, Barbara, 50733 Köln (DE)
(74) Representative: Roth, Andy Stefan
(86) International application number: PCT/EP2022/051492
(87) International publication number: WO 2022/167246

(56) References cited:
- WO-A1-2011/152782
- WO-A1-2017/046625

## Description

The present invention relates to soluble chelator compounds, methods for their production and their use for the modification and/or immobilization of target molecules.

### Background of the Invention

Chelation is a type of bonding of ions and molecules to metal ions. It involves the formation or presence of two or more separate coordinate bonds between a polydentate (multiple bonded) ligand and a single central atom.

These ligands are called chelants, chelators, chelating agents, or sequestering agents.

Chelation is useful in applications such as providing nutritional supplements, in chelation therapy to remove toxic metals from the body, as contrast agents in MRI scanning, in manufacturing using homogeneous catalysts, in chemical water treatment to assist in the removal of metals, and in fertilizers.

In recent years, chelators were also developed for use in protein purification and manipulation.

For example, a polyhistidine-tag, that is an amino acid motif in proteins that typically consists of at least six histidine (His) residues, is often attached to the N- or C-terminus of a protein. This His-tag is also known as "hexa histidine-tag, 6x His-tag, His-6 tag". The polyhistidine-tags is able to bind to metal ions and, thus, enables the binding of chelator to the tag. As such immobilized chelator may be used for affinity purification of genetically modified proteins and soluble chelator may be used for staining, certain purification methods, complex-building and other purposes.

However, prior art chelators, for example, such which rely on modified nitrilotriacetic acid (NTA), do not possess enough affinity in order to be used for a range of protein applications. This is on one hand because due to the rather weak binding the bond is lost is under stress, e.g. during applications with many washing steps and/or during gel electrophoresis. Furthermore, during protein production chelators are used which compete for binding with the chelator which should facilitate the desired function, such as for example immobilization or dye.

For example, tetradentate chelators, such as NTA, lose metal ions during purification, when other chelator, such as EDTA, or reductants, such as DTT, are present in the lysis buffer. EDTA is a very strong chelator, which removes membrane-stabilizing magnesium ions and is necessary for inhibiting metalloproteases in lysis buffer.

However, it removes almost all the metal ions from NTA resin if its concentration is high. Addition of DTT (dithiothreitol), which helps stabilizing the protein SH groups against oxidation to disulfides and protein dimerization, reduces metal ions, such as nickel, to the uncharged metal, so that the protein binding affinity is significantly lowered.

Thus, a great need exists in the field for stronger chelator, which can compete more effectively with the chelator already present during protein production.

The present invention presents soluble chelators, which are strong enough to compete with chelators, such as EDTA or DTT, which are present during many protein production methods.

In fact, the chelators of the present invention bind so strongly to metal ions, such as for example Ni, so that solutions of EDTA and/or DTT with a concentration, which are usual in the preparation and purification of recombinant proteins, do not dissolve or reduce the ion, as NTA does.

The soluble chelators of the present invention can be used for selective staining and labeling of his-tagged proteins in gels and on chromatography columns, as well as for selective functionalization of a his-tagged protein in solution by a dye, a nanoparticle, a tool for click chemistry, a radioactive molecule, or an enzymatic function.

Furthermore, the soluble chelators of the invention can also be used for expression control in preparation of a recombinant protein and for quantitative determination of proteins. In particular, the soluble chelators of the present invention show very high affinity, strong, tight assosiation and extremely slow dissociation, compared to the standard Ni-NTA, described in the prior art (e.g. EP0253303), and Tris NTA (e.g. described in WO 2006/013042), and allows its use as an alternative to antibodies in Western Blot or in affinity studies, e.g. by surface plasmon resonance.

WO 2017/046625 also discloses chelators forming complexes with target molecules, including recombinant proteins.

In one aspect, the invention pertains to a soluble chelator comprising at least one amino-carboxylic acid of the general structure as depicted in formula I: 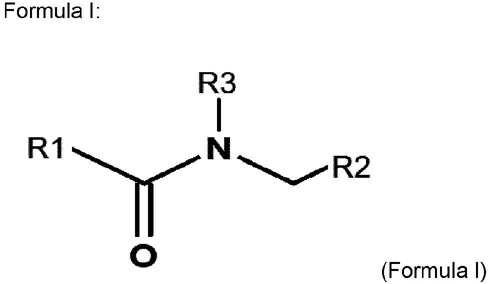 wherein **R1** is selected from a chelating carboxylic acid or a chelator-group, such as formula II: 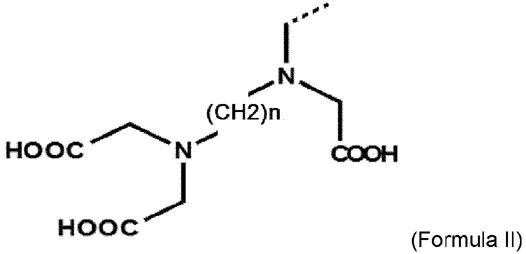 wherein n = 0, 1, 2, or 3;
or **R1** is selected from formula III: 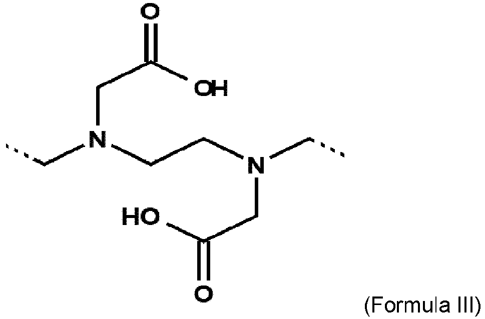 wherein each of the sides can be bound to R1;
and wherein
either
two molecules of formula I are bound to each other bivalently via **R2** resulting in a core-molecule of formula IV: 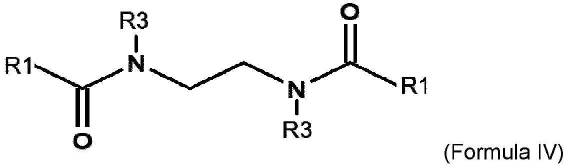 or
wherein three molecules of formula I are bound trivalent via **R2** to formula V: 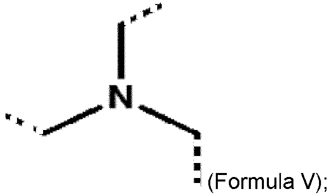 and wherein **R3** of the formulas mentioned above is selected either from the group comprising H, COOH, CH2-COOH and another molecule of formula I which is bound via R3-(CH₂)ₙ-R2, n = 0, 1, 2, or 3.

According to a definition of the IUPAC," chelator" means a polydentate ligand, which can be involved in formation of at least two coordinative bonds. In this invention the chelator are aminopolycarboxylic acids, such as EDTA (ethylenediamine tetraacetic acid), DTPA (diethylene triamine pentaacetic acid), TTHA (triethylenetetramine hexaacetic acid), EDDS (Ethylenediamine-N,N'-disuccinic acid), DOTA (1,4,7,10-tetraazacyclododecane-1,4,7,10-tetraacetic acid), NOTA (1,4,7-triazacyclononane-triacetic acid), and the like.

In the present invention, the carboxylic acid groups are written in the acid form, but with a pH between 6 and 8.5, which is common for the affinity purification of recombinant proteins, the biggest part of the carboxylic acid groups is in the carboxylate form, and some amino groups are protonated. In order to keep the description simple and clear, in the text and claims these groups are written as "COOH" and NH₂.

The chelator could contain other functional groups, such as carboxylic acids, alcohol, thiol, and the like as long as they do not interfere the functionality.

Examples for difunctional linkers are ethylene diamine, propylene diamine, diamino polyethylene glycol, and the like.

Examples for trifunctional linkers are 1,1,1-tris-(aminomethyl)-ethane, tris-(3-aminopropyl)-amine, tris-(2-aminoethyl)-amine, diethylenetriamine, 3-(2-aminoethyl)-pentane-1,5-diamine, and amino-functionalized trifunctional branched PEGs, basing on a glycerol core.

One possibility for the synthesis of the branched and linker-connected chelator is basing on an activated chelator, which can react with the amino groups of the linkers, so, e.g. EDTA monoanhydride or dianhydride. Alternatively, chelator and linker can be mixed in the ratio, which reflects the ratio in the resulting product, and coupling can be started by adding one or more condensing agents, such as carbodiimides, like EDC or DCC, or other reagents used in peptide syntheses.

After synthesis and coupling of the chelator chains, the reaction product can be charged with metal ions, which can bind the desired target proteins. As Ni, Cu, Co, Zn, Fe, Eu, Sc are suitable for reversible protein binding, nickel and cobalt are preferred for purification of His-tagged proteins, while iron and alumina are preferred for the isolation of phosphoproteins. For instance, the affinity of the chelator to the metal follows the sequence Cu > Ni > Zn > Co.

Thus, in another aspect, the soluble chelator comprises a positively charged metal-ion selected from the metals Ni, Co, Cu, Fe, Ca, Zn, Al, Eu, Ga, and Sc. Such positively charged metal-ions are for example: Ni⁺, Ni²⁺, Ni³⁺, Ni⁴⁺, Co²⁺, Cu²⁺, Cu³⁺, Fe²⁺, Fe³⁺, Ca²⁺, Zn²⁺, Pb²⁺, Al³⁺, Eu³⁺, Ga³⁺, Sc³⁺, Cr³⁺, Zr⁴⁺.

In another aspect, the soluble chelator comprises a number of carboxy groups of at least 8, at least 10, at least 12 and up to 14, up to 16.

In yet another aspect, the soluble chelator comprises a number of amino groups of at least 6, at least 8, at least 10, at least 12 and up to 14, up to 16.

In yet another aspect, the soluble chelator comprises R1 which is selected from the group comprising EDTA (ethylenediamine tetra-acetic acid), EGTA (ethylene glycol-bis-(β-aminoethyl ether)-N,N,N',N'-tetra-acetic acid), DTPA (diethylenetriamine penta-acetic acid), and EDDS (ethylenediamine-N,N'-di-succinic acid).

In yet another aspect, the soluble chelator is depicted in formula VI:

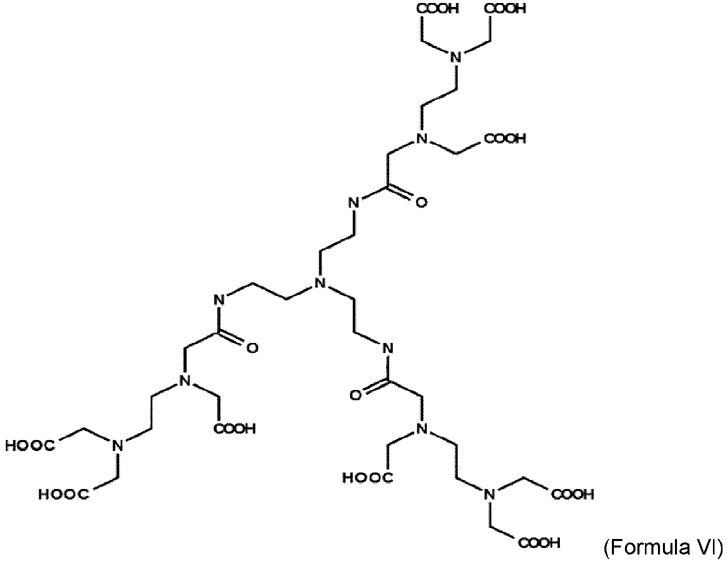

In yet another aspect, the soluble chelator is depicted in formula VII:

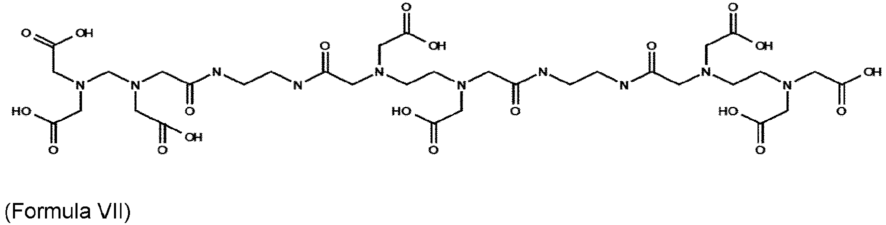

In yet another aspect, the soluble chelator is depicted in formula VIII:

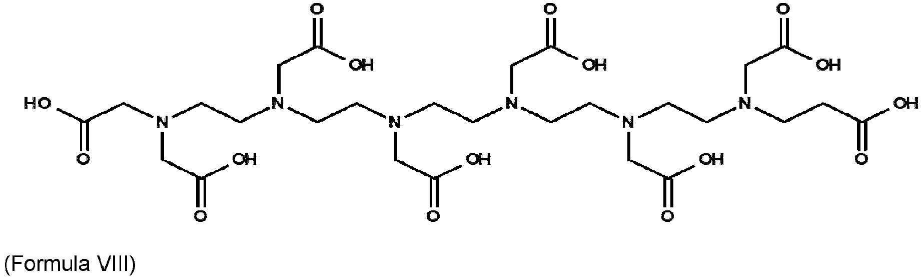

The chelators of formulae I to VIII can be functionalized by a number of modifications as described hereinunder.

In yet another aspect, the soluble chelator is further modified to comprise a tag, such as a biotin, desthiobiotin, a His-tag, a strep-tag, a FLAG-tag, a Rho-tag (e.g. Rho-1D4-tag).

In yet another aspect, the soluble chelator is further modified to comprise a dye, a colorant, a fluorophor, a nanoparticle, a functional group for click chemistry, a radioactive molecule, or a molecule with enzymatic activity. Per molecule one, two, or three molecules can be coupled. The chelator groups with the molecule attached are then reduced to a tetradental form.

In yet another aspect, the present invention also pertains to methods for producing the soluble chelator as mentioned herein, comprising the steps of:
a) Providing EDTA dianhydride and a trifunctional linker in a ratio of 4:1 to 2:1, preferably 2.75:1 to 3.25 to 1;
b) Coupling said dianhydride to said trifunctional linker via amine group onto the linker, wherein the linker reacts with three different dianhydrides, so that a substance (APA1)3-L is formed, wherein APA1 is EDTA with one anhydride group and one amide function, and L is the trifunctional linker;
c) Providing a molecule, which can be a dye, a colorant, a Fluorophor, a nanoparticle, a functional group for click chemistry, a radioactive molecule, or a molecule with enzyme activity, comprising one or more amine groups;
d) Coupling said linker-connected dianhydride to said molecule via amine group, wherein a single, two, or three carboxy groups per aminocarboxylic acid reacts with the amine moiety;
e) Providing water or an aqueous puffer to hydrolyze the remaining anhydride groups;
f) Immobilizing metal ions by contacting the solid phase-immobilized aminopolycarboxylic acid compound with solutions of metal ions, selected from Ni²⁺, Co²⁺, Cu²⁺, Zn²⁺, Al³⁺, Fe³⁺, Eu³⁺, Ga³⁺, Mn²⁺, Ca²⁺.

In yet another aspect, the present invention also pertains to methods for producing the soluble chelator as mentioned herein, comprising the steps of:
a) Providing EDTA monoanhydride and a trifunctional linker in a ratio of 4:1 to 2:1, preferably 2.75:1 to 3.25 to 1;
b) Coupling said monoanhydride to said trifunctional linker via amine group onto the linker, wherein the linker reacts with three different monoanhydrides, so that a substance (APA2)3-L is formed, wherein APA2 is EDTA with one amide function, and L is the trifunctional linker;
c) Providing a molecule, which can be a dye, a colorant, a Fluorophor, a nanoparticle, a functional group for click chemistry, a radioactive molecule, or a molecule with enzyme activity, comprising one or more amine groups;
d) Coupling said linker-connected EDTA to said molecule via amine group, wherein a single, two, or three carboxy groups per aminocarboxylic acid reacts with the amine moiety, by means of one or more condensing agents, such as carbodiimides, like EDC or DCC, or other reagents used in peptide syntheses;
e) Immobilizing metal ions by contacting the solid phase-immobilized aminopolycarboxylic acid compound with solutions of metal ions, selected from Ni²⁺, Co²⁺, Cu²⁺, Zn²⁺, Al³⁺, Fe³⁺, Eu³⁺, Ga³⁺, Mn²⁺, Ca²⁺.

In yet another aspect, the present invention also pertains to methods for producing the soluble chelator as mentioned herein, comprising the steps of:
a) Providing a solid phase resin, which is able to bind carboxylic acid groups, such as Wang resin, and EDTA, with two carboxylic acid protective groups, such as methyl ester or tert butylester;
b) Coupling said EDTA derivative to said solid phase resin by means of one or more condensing agents, such as carbodiimides, like EDC or DCC, or other reagents used in peptide syntheses;
c) Providing a trifunctional linker;
d) Coupling said trifunctional linker onto the remaining carboxylic acid group of the solid-phase bound EDTA;
e) Removal of the protective groups by trifluoroacetic acid, or the like (such as other aqueous acids);
f) Cleavage of the aminopolycaroxylic acid from the solid phase by trifluoroacetic acid, or the like;
g) Providing a molecule, which can be a dye, a colorant, a Fluorophor, a nanoparticle, a functional group for click chemistry, a radioactive molecule, or a molecule with enzyme activity, comprising one or more amine groups;
h) Coupling said linker-connected dianhydride to said molecule via amine group, wherein a single, two, or three carboxy groups per aminocarboxylic acid reacts with the amine moiety;
i) Immobilizing metal ions by contacting the solid phase-immobilized aminopolycarboxylic acid compound with solutions of metal ions, selected from Ni²⁺, Co²⁺, Cu²⁺, Zn²⁺, Al³⁺, Fe³⁺, Eu³⁺, Ga³⁺, Mn²⁺, Ca²⁺.

In yet another aspect, the present invention also pertains to uses of the soluble chelator of the present invention as a colorant, a dye, a fluorophor, a nanoparticle, as a tool for click-chemistry, a radioactive molecule or as a molecule with enzymatic function.

In yet another aspect, the present invention also pertains to the use of the soluble chelator for binding to a target protein comprising a tag which binds to metal ions, such as for example a polyhistidine-tag.

Polyhistidine-tags are common in molecular biology and can be used for separating recombinant proteins, expressed in bacteria, such as *E*. *coli,* yeast, and mammalia. Thus, His-tagged proteins bind with metal ion-loaded chelator at pH values, where the histidine groups are non-protonated, while the great majority of the remaining proteins do not interact with the metal ions. When the pH value is lowered and histidine becomes protonated, the interaction of the protein to the metal ion is cleared, and the protein can be separated from the metal ion and/or the chelate-complex. Alternatively, the separation can also be performed with applying high concentrations of imidazole, which binds onto the metal ions and replaces the his-tags, which leads to be separation of the protein from the metal ion and/or the chelate-complex. For this application, imidazole concentrations of 100 to 300 mM are commonly used.

In yet another aspect, the present invention also pertains to the use of the soluble chelator for complex formation.

In yet another aspect, the present invention also pertains to the use of the soluble chelator for detection of metal ions, preferably in combination with a His-tag or biotinylation.

In yet another aspect, the present invention also pertains to the use of the soluble chelator for binding onto functionalized streptavidin.

The soluble chelator of the present invention shows significant higher metal binding affinity as compared to conventional tetradentate chelators. For example, the metal binding capacity, determined with Cu²⁺, shows at least 750-2.000 µmol/mg, more preferred at least 800 - 1.500 µmol/mg. In addition to that, extremely high quantities of His-tagged proteins can be bound with the soluble chelator according to this invention. So, in the following examples up to 1 mmol protein per mg chelator can be bound by the soluble chelator of the invention.

The affinity of the soluble chelator of the present invention for His-tagged proteins is much higher, compared to the NTA analoga. So, immobilized for surface plasmon resonance measurements, there is no visible protein loss from the chip, while NTA and variants are showing protein leaching during wash. So, a stable, non-reversible protein binding via affinity bonds between metal and poly-His is feasible.

Moreover, because of the multimeric nature of the chelator, they are able to bind His-tagged proteins with a cooperative effect, which gives a much higher affinity than a monomeric chelator alone.

Thus, due to the strong affinity binding, the soluble chelator according to this invention show an extremely good resistance against solution-based chelating agents and reductants, e.g. even when 5 mM Imidazol, 10 mM Imidazol, or even 20 mM Imidazol are used the soluble chelator according to this invention maintain their protein-binding capacity. The same applies to protein-solutions comprising, alone or additionally to Imidazol, at least 5 mM, at least 10 mM, or even at least 20 mM EDTA; and/or at least 5 mM, at least 10 mM, or even at least 20 mM DTT. So, applications with e.g. proteins from eucaryotic systems are possible, such as SDS gel, blots, pull-downs, labelling of surface proteins in an expression, which cannot be done with conventional materials such as tris-NTA.

Due to the high resistance against chelator and reductants the soluble chelator according to this invention are particularly suitable for the binding to membrane proteins, especially GPCRs. For the membrane protein binding in many cases detergents, such as dodecyl maltoside, or n-tetradecyl phospho-choline (FOS-14) are used to stabilize the protein in aqueous solution against precipitation and denaturation. The soluble chelator according to this invention show high protein binding capacity and a good tolerance against said detergents.

Although the soluble chelator according to this invention is very suitable for protein-binding, also single-stranded nucleic acids may be bound selectively. This method is based upon reversible binding of accessible imidazyl moieties, as present in single-stranded nucleic acids, to metal ions. Such accessible imidazyl moieties are not present in double-stranded nucleic acids and, thus, a selective binding of the soluble chelator according to this invention to single-stranded nucleic acids may be used for example to separate single-stranded from double-stranded nucleic acids, for example by complexing, staining, etc. In one embodiment, such a selective binding may be used in a half-quantitive assay in order to tell single-stranded viral RNA within a sample (e.g. a sputum-sample of a patient).

Of course, the soluble chelator according to this invention is very suitable for the removal of metal ions from solutions. EDTA forms metal complexes with stability constants of about 14 to 25 (Mar-tell A.E. & Smith R.M. (1982), Critical Stability Constants, Vol. 5: First Supplement, Plenum Press, New York), so the soluble chelator according to this invention can efficiently be used to bind metal ions, such as Ni²⁺, Co²⁺, Mn²⁺, Cr³⁺, Pb²⁺, etc.. This may be applied during assays testing water quality with respect to metal-ion contamination and similar purposes. The content of metal ions, especially toxic and/or radioactive metals, in a solution may also be reduced by complexing the metal ions with the chelator of the present invention within the liquid and applying a purification step via a tag-moiety attached to the chelator. One example is to attach the soluble chelator according to this invention to magnetic particles, which can be added to a solution, mixed and removed by a magnetic separation after binding to metal ions.

### Embodiments

### Examples

### Example 1: Synthesis and functionalization of Tris-EDTA, linked by tris(2-aminoethyl)amine

### Synthesis of EDTA monoanhydride

30.0 g (117 mmol) EDTA dianhydride were dissolved in 200 ml DMSO under N₂ atmosphere. Then a mixture of 2.1 ml (117 mmol) water in 10 ml DMSO is added dropwise, and the mixture was stirred for three hours while a precipitate has formed. The mixture was cooled, suction-filtered and washed repeatedly with dry DMSO and diethyl ether. The precipitate was dried and used for the next steps.

### Reaction of EDTA monoanhydride with tris(2-aminoethyl)-amine

1 g (3.65 mmol) EDTA monoanhydride were dissolved in 30 ml DMSO under nitrogen, and a solution of ml tris(2-aminoethyl)-amine (mmol) in 10 ml DMSO was added dropwise. After the addition is finished, the suspension was stirred two hours at ambient temperature and three hours at 60 °C. The reaction mixture was evacuated under high vacuum, and the remaining product could be used for the next steps.

### Functionalization of the product with different fluorophors and nickel

5x 100 mg (5x108 micromol) of the reaction product are dissolved each in 50 ml water. Then 5x 25.0 mg of EDC (5x130 micromol), 5x 15.0 mg of N-hydroxy succinimide (5x130 micromol), and 69.3 mg (110 micromol) CY3-amine (Lumiprobe GmbH, Hannover, Germany), 47.0 mg (110 micromol) BDP-FL amine (Lumiprobe), 56.2 mg (110 micromol) FAM amine, 5 isomer (Lumiprobe), 90.4 mg (110 micromol) Atto 633 amine (Atto-Tec GmbH, Siegen, Germany), and 79.2 mg (110 micromol) Alpha Fluor^{™} 488 amine (AAT Bioquest Inc, Sunnyvale, CA, USA) were added, and the reaction mixture was stirred two hours. Then 105 mg (40 micromol) nickel sulfate hexahydrate were added to each tube, and the reaction mixture was stirred another two hours. The product was dried under high vacuum, washed several times with dry diethyl ether, and dried again. In the next step the conjugates were purified over a 5 ml PureCube Q Cartridge (Cube Biotech GmbH) and eluted with a 0 ech Gmb NaCl gradient using an FPLC system.

### Example 2: Synthesis and functionalization of a linear EDTA-EDA-EDTA-EDA-EDTA molecule

30.0 g (117 mmol) EDTA dianhydride were dissolved in 200 ml DMSO under N2 atmosphere. 5,21 ml (78 mmol) ethylene diamine were dissolved in 25 ml DMSO and added dropwise to the reaction mixture. After finishing the addition, the mixture was stirred two hours at ambient temperature, and three hours at 60 °C. Then the reaction mixture was evacuated under high vacuum, and the remaining product could be used for the next steps.

### Functionalization of the product with different fluorophors and nickel

5x 100 mg (5x108 micromol) of the reaction product are dissolved each in 50 ml water. Then 5x 25.0 mg of EDC (5x130 micromol), 5x 15.0 mg of N-hydroxy succinimide (5x130 micromol), and 69.3 mg (110 micromol) CY3-amine (Lumiprobe GmbH, Hannover, Germany), 47.0 mg (110 micromol) BDP-FL amine (Lumiprobe), 56.2 mg (110 micromol) FAM amine, 5 isomer (Lumiprobe), 90.4 mg (110 micromol) Atto 633 amine (Atto-Tec GmbH, Siegen, Germany), and 79.2 mg (110 micromol) Alpha Fluor^{™} 488 amine (AAT Bioquest Inc, Sunnyvale, CA, USA) were added, and the reaction mixture was stirred two hours. Then 105 mg (40 micromol) nickel sulfate hexahydrate were added to each tube, and the reaction mixture was stirred another two hours. The product was dried under high vacuum, washed several times with dry diethyl ether, and dried again. In the next step the conjugates were purified over a 5 ml PureCube Q Cartridge (Cube Biotech GmbH) and eluted with a 0 - 2.5 M NaCl gradient using an FPLC system.

### Example 3: Solid Phase Synthesis and functionalization of Tris-EDTA, linked by tris-(2-aminoethyl)-amine

### Coupling of EDTA-bis-methylester onto Wang Resin

In a round-bottom flask 0.5g Wang resin (Merck Millipore, Darmstadt, Germany) is covered with DMF and allowed to swell for 30 min. In another round-bottom flask 2 g EDTA-bis-methyl ester (preparation described in Govender, Journal of Membrane Science 279 (2006) 120-128) is dissolved in dry Dichloromethane under argon atmosphere. A small amount of DMF is added to achieve complete dissolution. The solution is cooled to 0 °C and 1 g N, N'-Diisopropylcarbodiimide is slowly added. The mixture is stirred for 20 min at the same temperature followed by evaporation. The residue is dissolved in 5 ml DMF and added to the resin suspension followed by addition of 100 mg 4-Dimethylaminopyridine. The suspension is shaken at room temperature for 1 h. For capping the rest of the free hydroxyl groups, 250 µl of acetic anhydride and 500 µl pyridine are added to the reaction mixture. The mixture is shaken at room temperature for additional 30 min. Resin is filtered, washed with DMF (3x), DCM (3x), MeOH (3x) and dried.

### Coupling of Tris-aminoethyl-amin

The functionalized Wang Resin is resuspended in 5 m DMF, and after 30 minutes 1 ml tris-(2-aminoethyl)-amine), 1.5 g 2-(1H-Benzotriazol-1-yl)-1,1,3,3-tetramethyluronium tetrafluoroborate (TBTU, Sigma Aldrich, Schnelldorf, Germany) and 2 g ethyl-diisopropylamine in 5 ml DMF are added. After 60 minutes incubation the product, the resin is suction-filtered and washed five times with DMF.

### Second Coupling of EDTA-bis-methyl ester

The functionalized Wang Resin is resuspended in 5 m DMF, and after 30 minutes 1.5 g EDTA-bis-t-butyl ester, 1.5 g TBTU, and 3 ml ethyl-diisopropylamine in 5 ml DMF are added. After 60 minutes incubation the product, the resin is suction-filtered and washed five times with DMF. Then the product is washed by dichloromethane and methanol and dried under vacuum.

### Cleavage from Solid Phase, Removal of Methyl Protective Groups

The Wang resin is transferred to a sintered glass funnel with fine porosity and washed three times with DMF, then five times with dichloromethane. The resin is resuspended in 50%TFA in DCM (v/v), and the mixture is stirred for three hours. Now the resin is filtered and washed three times with small portions of TFA. The filtrates are combined, and 100 ml cold diethyl ether is added to precipitate the peptide. The peptide is filtered through a fine sintered glass funnel, washed with cold ether, and dried.

## Claims

1. A soluble chelator comprising at least one amino-carboxylic acid of the general structure as depicted in **formula I**: 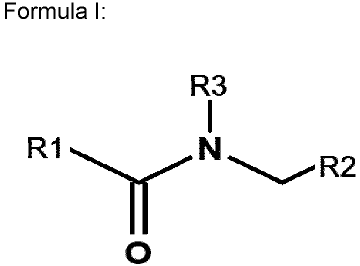
wherein **R1** is selected from a chelating carboxylic acid or a chelator-group, such as **formula II**: 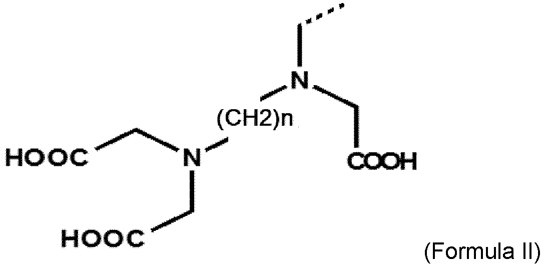
wherein n = 0, 1, 2, or 3;
or **R1** is selected from **formula III**: 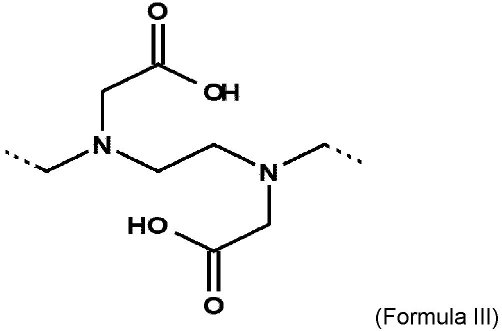
wherein each of the sides can be bound to **R1**; and
wherein
**either**
two molecules of **formula I** are bound to each other bivalently via **R2** resulting in a core-molecule of formula IV: 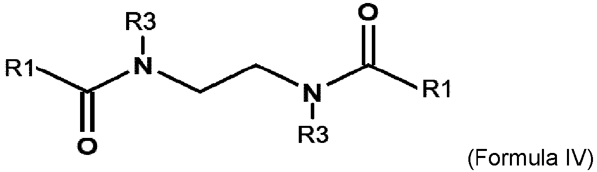 **or**
wherein three molecules of **formula I** are bound trivalent via **R2** to **formula V**: 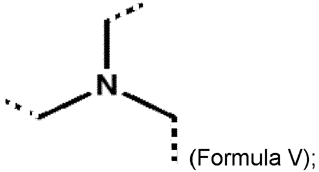 ; and
wherein **R3** of the formulas above is selected either from the group comprising H, COOH, CH₂-COOH and another molecule of formula I which is bound via **R3**-(CH₂)ₙ-**R2**, n = 0, 1, 2, or 3.

2. The soluble chelator of claim 1 comprising a positively charged metal-ion selected from the metals Ni, Co, Cu, Fe, Ca, Zn, Al, Eu, Ga, and Sc.

3. The soluble chelator of any of claims 1 or 2, wherein the number of carboxy groups is at least 8.

4. The soluble chelator of any of claims 1 to 3, wherein the number of amino groups is at least 6.

5. The soluble chelator of any of the previous claims wherein R1 is selected from the group comprising EDTA (ethylene diamine tetra acetic acid), EGTA (ethylene glycol-bis-(β-aminoethyl ether)-N,N,N',N'-tetra acetic acid), DTPA (diethylene triamine penta acetic acid), and EDDS (ethylene diamine-N,N'-di succinic acid).

6. The soluble chelator of claim 1 as depicted in formula VI: 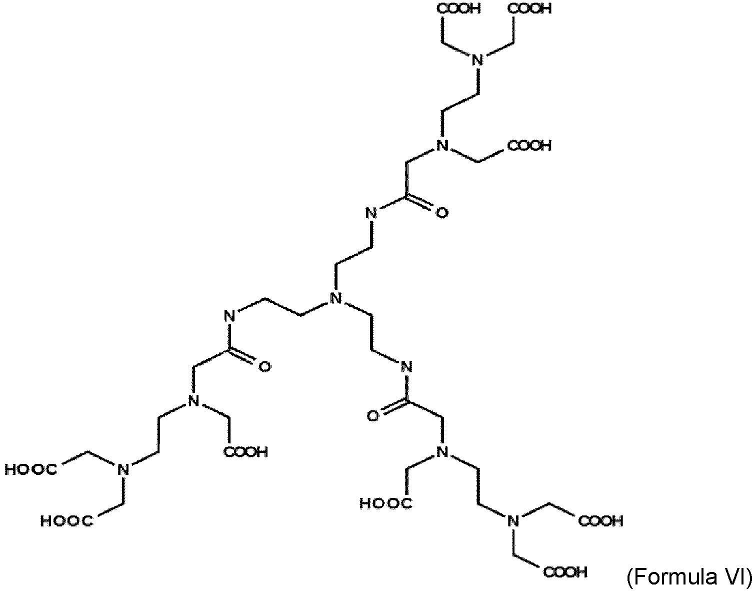
or as depicted in formula VII: 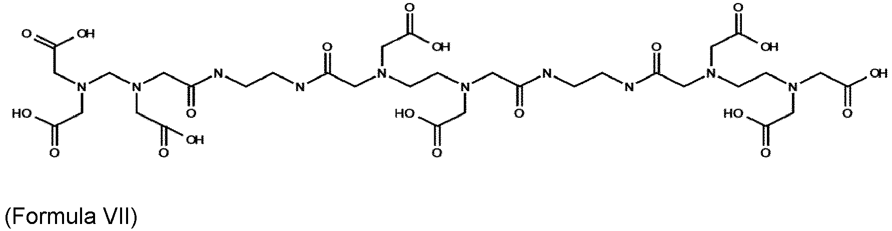
or as depicted in formula VIII:

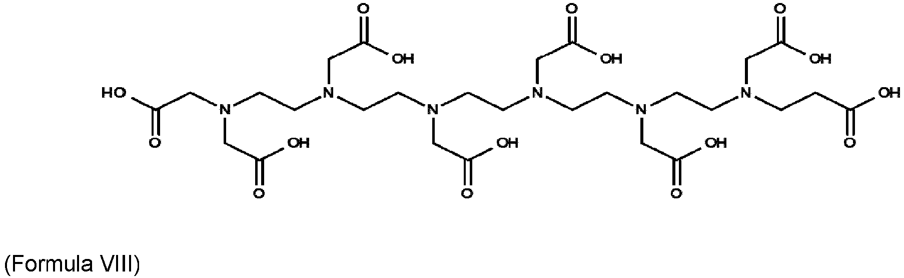

7. The soluble chelator of any of the previous claims, wherein the chelator is further modified to comprise a tag, such as a biotinylation, a His-tag, a strep-tag, a FLAG-tag, a Rho-tag (e.g. Rho-1D4-tag).

8. Method for producing the soluble, chelators of any of the previous claims, comprising the steps of:
a. Providing EDTA dianhydride and a trifunctional linker in a ratio of 4:1 to 2:1, preferably 2.75:1 to 3.25 to 1;
b. Coupling said dianhydride to said trifunctional linker via amine group onto the linker, wherein the linker reacts with three different dianhydrides, so that a substance (APA1)3-L is formed, wherein APA1 is EDTA with one anhydride group and one amide function, and L is the trifunctional linker;
c. Providing a molecule, which can be a dye, a colorant, a fluorophor, a nanoparticle, a functional group for click chemistry, a radioactive molecule, or a molecule with enzyme activity, comprising one or more amine groups;
d. Coupling said linker-connected dianhydride to said molecule via amine group, wherein a single, two, or three carboxy groups per aminocarboxylic acid reacts with the amine moiety;
e. Providing water or an aqueous puffer to hydrolyze the remaining anhydride groups;
f. Immobilizing metal ions by contacting the solid phase-immobilized aminopolycarboxylic acid compound with solutions of metal ions, selected from Ni²⁺, Co²⁺, Cu²⁺, Zn²⁺, Al³⁺, Fe³⁺, Eu³⁺, Ga³⁺, Mn²⁺, Ca²⁺.

9. Method for producing the soluble chelators of any of the previous claims, comprising the steps of:
g. Providing EDTA monoanhydride and a trifunctional linker in a ratio of 4:1 to 2:1, preferably 2.75:1 to 3.25 to 1;
h. Coupling said monoanhydride to said trifunctional linker via amine group onto the linker, wherein the linker reacts with three different monoanhydrides, so that a substance (APA2)3-L is formed, wherein APA2 is EDTA with one amide function, and L is the trifunctional linker;
i. Providing a molecule, which can be a dye, a colorant, a fluorophor, a nanoparticle, a functional group for click chemistry, a radioactive molecule, or a molecule with enzyme activity, comprising one or more amine groups;
j. Coupling said linker-connected EDTA to said molecule via amine group, wherein a single, two, or three carboxy groups per aminocarboxylic acid reacts with the amine moiety, by means of one or more condensing agents, such as carbodiimides, like EDC or DCC, or other reagents used in peptide syntheses;
k. Immobilizing metal ions by contacting the solid phase-immobilized aminopolycarboxylic acid compound with solutions of metal ions, selected from Ni²⁺, Co²⁺, Cu²⁺, Zn²⁺, Al³⁺, Fe³⁺, Eu³⁺, Ga³⁺, Mn²⁺, Ca²⁺.

10. Method for producing the soluble chelators of any of the previous claims, comrising the steps of:
I. Providing a solid phase resin, which is able to bind carboxylic acid groups, such as Wang resin, and EDTA, with two carboxylic acid protective groups, such as methyl ester or tert butylester;
m. Coupling said EDTA derivative to said solid phase resin by means of one or more condensing agents, such as carbodiimides, like EDC or DCC, or other reagents used in peptide syntheses;
n. Providing a trifunctional linker;
o. Coupling said trifunctional linker onto the remaining carboxylic acid group of the solid-phase bound EDTA;
p. Removal of the protective groups by trifluoroacetic acid, or the like;
q. Cleavage of the aminopolycaroxylic acid from the solid phase by trifluoroacetic acid, or the like;
r. Providing a molecule, which can be a dye, a colorant, a fluorophor, a nanoparticle, a functional group for click chemistry, a radioactive molecule, or a molecule with enzyme activity, comprising one or more amine groups;
s. Coupling said linker-connected dianhydride to said molecule via amine group, wherein a single, two, or three carboxy groups per aminocarboxylic acid reacts with the amine moiety;
t. Immobilizing metal ions by contacting the solid phase-immobilized aminopolycarboxylic acid compound with solutions of metal ions, selected from Ni²⁺, Co²⁺, Cu²⁺, Zn²⁺, Al³⁺, Fe³⁺, Eu³⁺, Ga³⁺, Mn²⁺, Ca²⁺.

11. Method for producing the soluble chelators of any of the previous claims, comprising the steps of: Use of the soluble, chelators of any of the previous claims as a colorant, a dye, a fluorophor, a nanoparticle, as a tool for click-chemistry, a radioactive molecule or as a molecule with enzymatic function.

12. Use of the soluble chelators of any of the previous claims for binding to a target protein comprising a tag which binds to metal ions, such as for example a polyhistidine-tag, for complex formation, for detection of metal ions, preferably in combination with a His-tag or biotinylation, or for binding onto functionalized streptavidin.

## Patentansprüche

1. Löslicher Chelatbildner, der mindestens eine Aminocarbonsäure mit der allgemeinen Struktur umfasst, wie in **Formel I** verbildlicht: 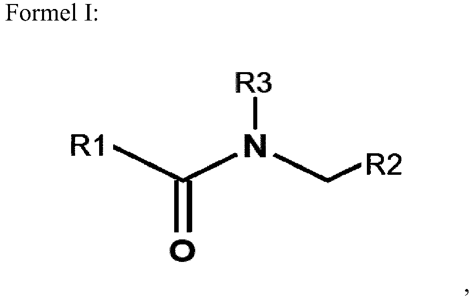
wobei **R1** ausgewählt ist aus einer chelatbildenden Carbonsäure oder einer Chelatbildnergruppe, wie **Formel II**: 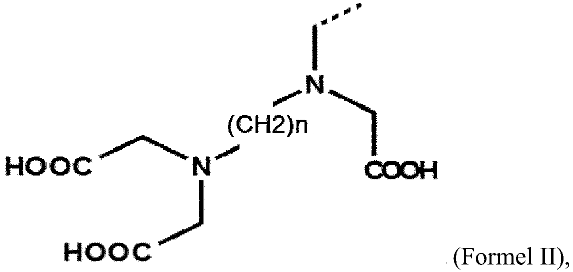
wobei n = 0, 1, 2 oder 3;
oder **R1** ausgewählt ist aus **Formel III**: 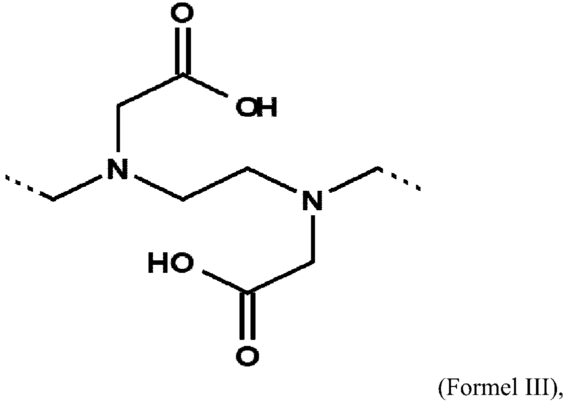
wobei jede der Seiten an **R1** gebunden werden kann; und
wobei
**entweder**
zwei Moleküle von **Formel I** über **R2** bivalent aneinander gebunden sind, was zu einem Kernmolekül von **Formel IV** führt: 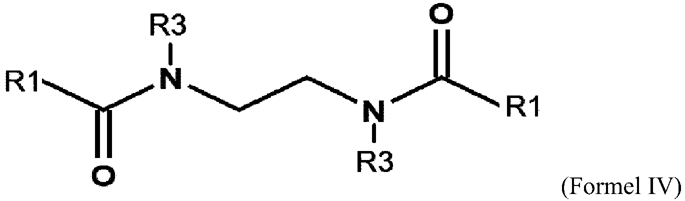 **oder**
wobei drei Moleküle von **Formel I** über **R2** dreiwertig an **Formel V** gebunden sind: 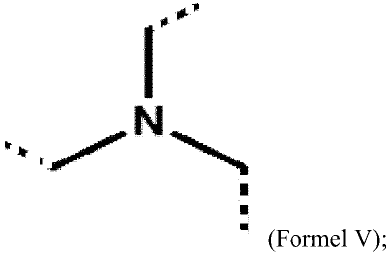 und
wobei **R3** der obigen Formeln entweder aus der Gruppe ausgewählt ist, die H, COOH, CH₂-COOH und ein anderes Molekül von Formel I umfasst, das über **R3**-(CH₂)ₙ-**R2**, n = 0, 1, 2 oder 3, gebunden ist.

2. Löslicher Chelatbildner nach Anspruch 1, umfassend ein positiv geladenes Metallion, ausgewählt aus den Metallen Ni, Co, Cu, Fe, Ca, Zn, Al, Eu, Ga und Sc.

3. Löslicher Chelatbildner nach einem der Ansprüche 1 oder 2, wobei die Anzahl der Carboxygruppen mindestens 8 beträgt.

4. Löslicher Chelatbildner nach einem der Ansprüche 1 bis 3, wobei die Anzahl der Aminogruppen mindestens 6 beträgt.

5. Löslicher Chelatbildner nach einem der vorhergehenden Ansprüche, wobei R1 ausgewählt ist aus der Gruppe umfassend EDTA (Ethylendiamintetraessigsäure), EGTA (Ethylenglycol-bis-(β-aminoethylether)-N,N,N',N'-tetraessigsäure), DTPA (Diethylentriaminpentaessigsäure) und EDDS (Ethylendiamin-N,N'-dibernsteinsäure).

6. Löslicher Chelatbildner nach Anspruch 1, wie in Formel VI verbildlicht: 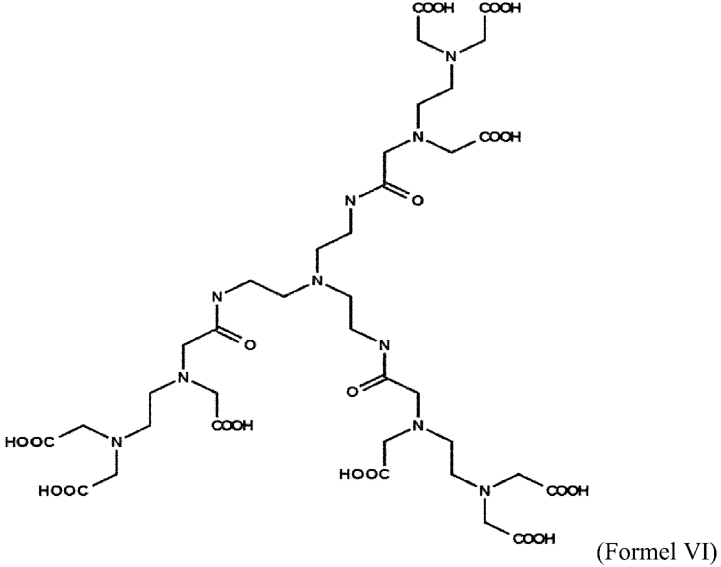
oder wie in Formel VII verbildlicht: 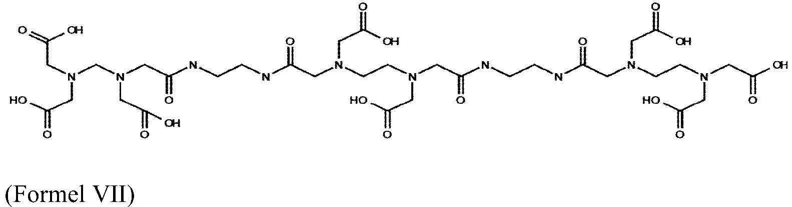
oder wie in Formel VIII verbildlicht:

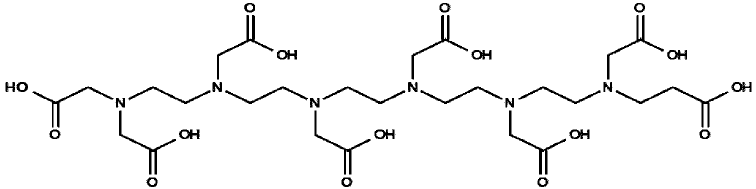

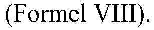

7. Löslicher Chelatbildner nach einem der vorhergehenden Ansprüche, wobei der Chelatbildner ferner so modifiziert ist, dass er eine Markierung, wie eine Biotinylierung, eine His-Markierung, eine Strep-Markierung, eine FLAG-Markierung, eine Rho-Markierung (z. B. Rho-1D4-Markierung) umfasst.

8. Verfahren zur Herstellung der löslichen Chelatbildner nach einem der vorhergehenden Ansprüche, umfassend die folgenden Schritte:
a. Bereitstellen von EDTA-Dianhydrid und einem trifunktionalen Linker in einem Verhältnis von 4:1 bis 2:1, vorzugsweise 2,75:1 bis 3,25:1;
b. Koppeln des Dianhydrids an den trifunktionalen Linker über eine Amingruppe an den Linker, wobei der Linker mit drei verschiedenen Dianhydriden reagiert, sodass eine Substanz (APA1)3-L gebildet wird, wobei APA1 EDTA mit einer Anhydridgruppe und einer Amidfunktion ist und L der trifunktionale Linker ist;
c. Bereitstellen eines Moleküls, das ein Farbstoff, ein Färbemittel, ein Fluorophor, ein Nanopartikel, eine funktionelle Gruppe für die Click-Chemie, ein radioaktives Molekül oder ein Molekül mit Enzymaktivität sein kann, umfassend eine oder mehrere Amingruppen;
d. Koppeln des über einen Linker verbundenen Dianhydrids an das Molekül über eine Amingruppe, wobei eine einzelne, zwei oder drei Carboxygruppen pro Aminocarbonsäure mit dem Aminanteil reagieren;
e. Bereitstellen von Wasser oder eines wässrigen Puffers zum Hydrolysieren der verbleibenden Anhydridgruppen;
f. Immobilisieren von Metallionen durch Inkontaktbringen der festphasen-immobilisierten Aminopolycarbonsäureverbindung mit Lösungen von Metallionen, ausgewählt aus Ni²⁺, Co²⁺, Cu²⁺, Zn²⁺, Al³⁺, Fe³⁺, Eu³⁺, Ga³⁺, Mn²⁺, Ca²⁺.

9. Verfahren zur Herstellung der löslichen Chelatbildner nach einem der vorhergehenden Ansprüche, umfassend die folgenden Schritte:
g. Bereitstellen von EDTA-Monoanhydrid und einem trifunktionalen Linker in einem Verhältnis von 4:1 bis 2:1, vorzugsweise 2,75:1 bis 3,25:1;
h. Koppeln des Monoanhydrids an den trifunktionalen Linker über eine Amingruppe an den Linker, wobei der Linker mit drei verschiedenen Monoanhydriden reagiert, sodass eine Substanz (APA2)3-L gebildet wird, wobei APA2 EDTA mit einer Amidfunktion ist und L der trifunktionale Linker ist;
i. Bereitstellen eines Moleküls, das ein Farbstoff, ein Färbemittel, ein Fluorophor, ein Nanopartikel, eine funktionelle Gruppe für die Click-Chemie, ein radioaktives Molekül oder ein Molekül mit Enzymaktivität sein kann, umfassend eine oder mehrere Amingruppen;
j. Koppeln des über einen Linker verbundenen EDTA an das Molekül über eine Amingruppe, wobei eine einzelne, zwei oder drei Carboxygruppen pro Aminocarbonsäure mit dem Aminanteil reagieren, mittels eines oder mehrerer Kondensationsmittel, wie Carbodiimiden, wie EDC oder DCC, oder anderer in Peptidsynthesen verwendeter Reagenzien;
k. Immobilisieren von Metallionen durch Inkontaktbringen der festphasen-immobilisierten Aminopolycarbonsäureverbindung mit Lösungen von Metallionen, ausgewählt aus Ni²⁺, Co²⁺, Cu²⁺, Zn²⁺, Al³⁺, Fe³⁺, Eu³⁺, Ga³⁺, Mn²⁺, Ca²⁺.

10. Verfahren zur Herstellung der löslichen Chelatbildner nach einem der vorhergehenden Ansprüche, umfassend die folgenden Schritte:
1. Bereitstellen eines Festphasenharzes, das in der Lage ist, Carbonsäuregruppen zu binden, wie Wang-Harz und EDTA, mit zwei Carbonsäureschutzgruppen, wie Methylester oder tert-Butylester;
m. Koppeln des EDTA-Derivats an das Festphasenharz mittels eines oder mehrerer Kondensationsmittel, wie Carbodiimide, wie EDC oder DCC, oder anderer in Peptidsynthesen verwendeter Reagenzien;
n. Bereitstellen eines trifunktionalen Linkers;
o. Koppeln des trifunktionalen Linkers an die verbleibende Carbonsäuregruppe des festphasen-gebundenen EDTA;
p. Entfernen der Schutzgruppen durch Trifluoressigsäure oder dergleichen;
q. Abspaltung der Aminopolycarbonsäure aus der Festphase durch Trifluoressigsäure oder dergleichen;
r. Bereitstellen eines Moleküls, das ein Farbstoff, ein Färbemittel, ein Fluorophor, ein Nanopartikel, eine funktionelle Gruppe für die Click-Chemie, ein radioaktives Molekül oder ein Molekül mit Enzymaktivität sein kann, umfassend eine oder mehrere Amingruppen;
s. Koppeln des über einen Linker verbundenen Dianhydrids an das Molekül über eine Amingruppe, wobei eine einzelne, zwei oder drei Carboxygruppen pro Aminocarbonsäure mit dem Aminanteil reagieren;
t. Immobilisieren von Metallionen durch Inkontaktbringen der festphasen-immobilisierten Aminopolycarbonsäureverbindung mit Lösungen von Metallionen, ausgewählt aus Ni²⁺, Co²⁺, Cu²⁺, Zn²⁺, Al³⁺, Fe³⁺, Eu³⁺, Ga³⁺, Mn²⁺, Ca²⁺.

11. Verfahren zur Herstellung der löslichen Chelatbildner nach einem der vorhergehenden Ansprüche, umfassend die folgenden Schritte: Verwendung der löslichen Chelatbildner nach einem der vorhergehenden Ansprüche als ein Farbstoff, ein Färbemittel, ein Fluorophor, ein Nanopartikel, als ein Werkzeug für die Click-Chemie, ein radioaktives Molekül oder als ein Molekül mit enzymatischer Funktion.

12. Verwendung der löslichen Chelatbildner nach einem der vorhergehenden Ansprüche zur Bindung an ein Zielprotein, das eine Markierung umfasst, die an Metallionen bindet, wie beispielsweise eine Polyhistidin-Markierung, zur Komplexbildung, zum Nachweis von Metallionen, vorzugsweise in Kombination mit einer His-Markierung oder Biotinylierung, oder zur Bindung an funktionalisiertes Streptavidin.

## Revendications

1. Chélateur soluble comprenant au moins un acide amino-carboxylique de structure générale telle que représentée par la **formule I** : 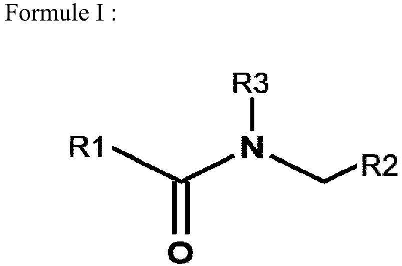
**R1** étant choisi parmi un acide carboxylique chélatant ou un groupe chélateur, tel que la **formule II** : 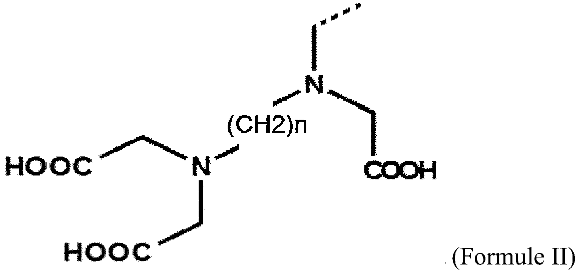
où n = 0, 1, 2 ou 3
ou **R1** est choisi parmi la **formule III** : 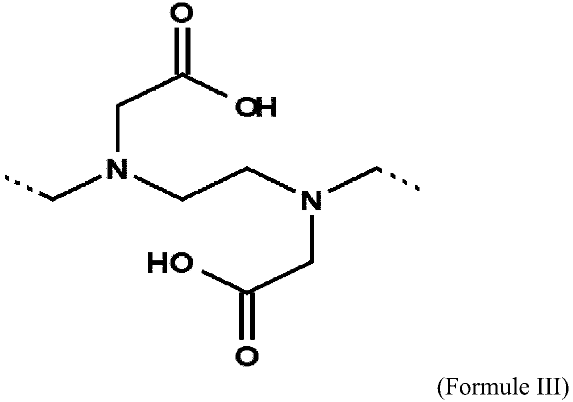
chacun des côtés pouvant être lié à **R1** ; et où
**soit**
deux molécules de **formule** I sont liées l'une à l'autre de manière bivalente par l'intermédiaire de **R2**, ce qui donne une molécule noyau de formule IV : 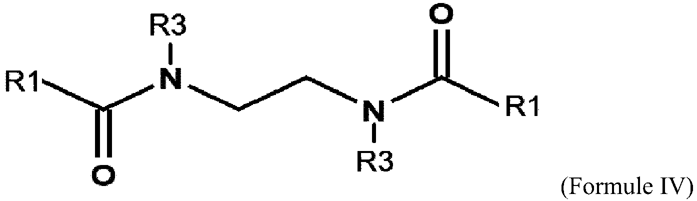 **soit**
trois molécules de **formule** I étant liées de manière trivalente par l'intermédiaire de **R2** à la **formule V** : 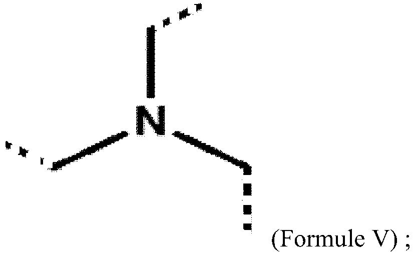 et
**R3** des formules ci-dessus étant choisi dans le groupe comprenant H, COOH, CH₂-COOH et une autre molécule de formule I qui est liée par l'intermédiaire de **R3**-(CH₂)ₙ-**R2**, n = 0, 1, 2 ou 3.

2. Chélateur soluble de la revendication 1 comprenant un ion métallique chargé positivement choisi parmi les métaux Ni, Co, Cu, Fe, Ca, Zn, Al, Eu, Ga et Sc.

3. Chélateur soluble de l'une quelconque des revendications 1 ou 2, dans lequel le nombre de groupes carboxy est d'au moins 8.

4. Chélateur soluble de l'une quelconque des revendications 1 à 3, dans lequel le nombre de groupes amino est d'au moins 6.

5. Chélateur soluble de l'une quelconque des revendications précédentes, dans lequel R1 est choisi dans le groupe comprenant l'EDTA (acide éthylène diamine tétra-acétique), l'EGTA (acide éthylène glycol-bis-(β-aminoéthyléther)-N,N,N',N'-tétra-acétique), le DTPA (acide diéthylène triamine penta-acétique) et l'EDDS (acide éthylène diamine-N,N'-di-succinique).

6. Chélateur soluble de la revendication 1 tel que représenté par la formule VI : 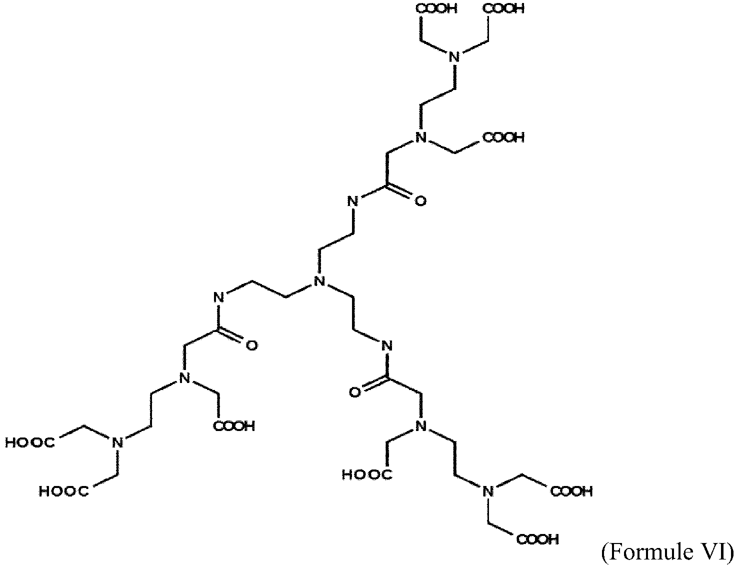
ou tel que représenté par la formule VII : 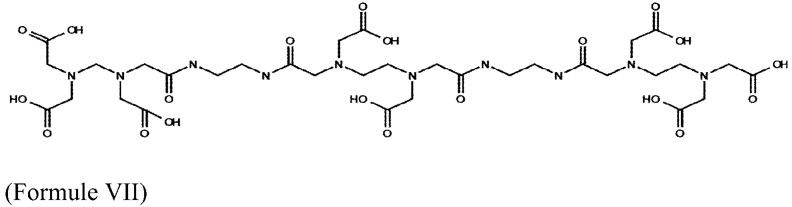
ou tel que représenté par la formule VIII :

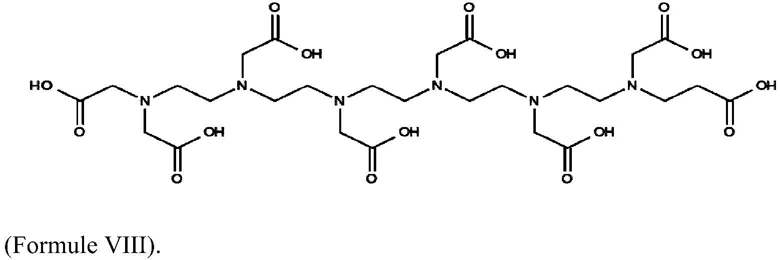

7. Chélateur soluble de l'une quelconque des revendications précédentes, ledit chélateur étant en outre modifié pour comprendre une étiquette, telle qu'une biotinylation, une étiquette His, une étiquette strep, une étiquette FLAG, une étiquette Rho (par exemple, une étiquette Rho-1D4).

8. Procédé permettant la production des chélateurs solubles de l'une quelconque des revendications précédentes, comprenant les étapes de :
a. fourniture de dianhydride d'EDTA et d'un coupleur trifonctionnel dans un rapport de 4:1 à 2:1, de préférence de 2,75:1 à 3,25:1 ;
b. couplage dudit dianhydride audit coupleur trifonctionnel par l'intermédiaire d'un groupe amine sur le coupleur, ledit coupleur réagissant avec trois dianhydrides différents, de sorte qu'une substance (APA1)3-L soit formée, ladite substance APA1 étant l'EDTA comportant un groupe anhydride et une fonction amide, et L étant le coupleur trifonctionnel ;
c. fourniture d'une molécule, qui peut être une teinture, un colorant, un fluorophore, une nanoparticule, un groupe fonctionnel pour la chimie clic, une molécule radioactive ou une molécule à activité enzymatique, comprenant un ou plusieurs groupes amine ;
d. couplage dudit dianhydride relié au coupleur à ladite molécule par l'intermédiaire d'un groupe amine, un seul, deux ou trois groupes carboxy par acide aminocarboxylique réagissant avec la fraction amine ;
e. fourniture d'eau ou d'un tampon aqueux pour hydrolyser les groupes anhydrides restants ;
f. immobilisation d'ions métalliques en mettant en contact le composé d'acide amino-polycarboxylique immobilisé sur phase solide avec des solutions d'ions métalliques, choisis parmi Ni²⁺, Co²⁺, Cu²⁺, Zn²⁺, Al³⁺, Fe³⁺, Eu³⁺, Ga³⁺, Mn²⁺, Ca²⁺.

9. Procédé permettant la production des chélateurs solubles de l'une quelconque des revendications précédentes, comprenant les étapes de :
g. fourniture de monoanhydride d'EDTA et d'un coupleur trifonctionnel dans un rapport de 4:1 à 2:1, de préférence de 2,75:1 à 3,25:1 ;
h. couplage dudit monoanhydride audit coupleur trifonctionnel par l'intermédiaire d'un groupe amine sur le coupleur, ledit coupleur réagissant avec trois monoanhydrides différents, de sorte qu'une substance (APA2)3-L soit formée, ladite substance APA2 étant l'EDTA comportant une fonction amide, et L étant le coupleur trifonctionnel ;
i. fourniture d'une molécule, qui peut être une teinture, un colorant, un fluorophore, une nanoparticule, un groupe fonctionnel pour la chimie clic, une molécule radioactive ou une molécule à activité enzymatique, comprenant un ou plusieurs groupes amine ;
j. couplage dudit EDTA relié au coupleur à ladite molécule par l'intermédiaire d'un groupe amine, un seul, deux ou trois groupes carboxy par acide aminocarboxylique réagissant avec la fraction amine, au moyen d'un ou plusieurs agents de condensation, tels que des carbodiimides, comme l'EDC ou le DCC, ou d'autres réactifs utilisés dans des synthèses peptidiques ;
k. immobilisation d'ions métalliques en mettant en contact le composé d'acide amino-polycarboxylique immobilisé sur phase solide avec des solutions d'ions métalliques, choisis parmi Ni²⁺, Co²⁺, Cu²⁺, Zn²⁺, Al³⁺, Fe³⁺, Eu³⁺, Ga³⁺, Mn²⁺, Ca²⁺.

10. Procédé permettant la production des chélateurs solubles de l'une quelconque des revendications précédentes, comprenant les étapes de :
1. fourniture d'une résine en phase solide, qui est capable de lier des groupes acide carboxylique, tels que la résine Wang, et d'EDTA, avec deux groupes protecteurs d'acide carboxylique, tels que l'ester méthylique ou l'ester tert-butylique ;
m. couplage dudit dérivé d'EDTA à ladite résine en phase solide au moyen d'un ou plusieurs agents de condensation, tels que des carbodiimides, comme l'EDC ou le DCC, ou d'autres réactifs utilisés dans des synthèses peptidiques ;
n. fourniture d'un coupleur trifonctionnel ;
o. couplage dudit coupleur trifonctionnel sur le groupe acide carboxylique restant de l'EDTA lié à la phase solide ;
p. élimination des groupes protecteurs par l'acide trifluoroacétique, ou d'autres analogues ;
q. clivage de l'acide amino-polycarboxylique de la phase solide par l'acide trifluoroacétique, ou d'autres analogues ;
r. fourniture d'une molécule, qui peut être une teinture, un colorant, un fluorophore, une nanoparticule, un groupe fonctionnel pour la chimie clic, une molécule radioactive ou une molécule à activité enzymatique, comprenant un ou plusieurs groupes amine ;
s. couplage dudit dianhydride relié au coupleur à ladite molécule par l'intermédiaire d'un groupe amine, un seul, deux ou trois groupes carboxy par acide aminocarboxylique réagissant avec la fraction amine ;
t. immobilisation d'ions métalliques en mettant en contact le composé d'acide amino-polycarboxylique immobilisé sur phase solide avec des solutions d'ions métalliques, choisis parmi Ni²⁺, Co²⁺, Cu²⁺, Zn²⁺, Al³⁺, Fe³⁺, Eu³⁺, Ga³⁺, Mn²⁺, Ca²⁺.

11. Procédé permettant la production des chélateurs solubles de l'une quelconque des revendications précédentes, comprenant les étapes de : utilisation des chélateurs solubles de l'une quelconque des revendications précédentes en tant que colorant, teinture, fluorophore, nanoparticule, en tant qu'outil pour la chimie clic, en tant que molécule radioactive ou en tant que molécule à fonction enzymatique.

12. Utilisation des chélateurs solubles de l'une quelconque des revendications précédentes pour la liaison à une protéine cible comprenant une étiquette qui se lie à des ions métalliques, telle que par exemple une étiquette polyhistidine, pour la formation de complexes, pour la détection d'ions métalliques, de préférence en combinaison avec une étiquette His ou une biotinylation, ou pour la liaison à la streptavidine fonctionnalisée.
